# EUROPEAN PATENT SPECIFICATION

(11) **EP 3 801 263 B1**
(45) Date of publication and mention of the grant of the patent: **24.01.2024**
(21) Application number: 18919992.0
(22) Date of filing: 25.05.2018
(51) Int. Cl.: A61B 5/151, A61B 5/15

(54) **CAPILLARY BLOOD COLLECTION DEVICE**
KAPILLARBLUTENTNAHMEVORRICHTUNG
DISPOSITIF DE PRÉLÈVEMENT DE SANG CAPILLAIRE

(43) Date of publication of application: 14.04.2021
(73) Proprietor: Aiki Riotech Corporation, Inazawa-shi, Aichi 492-8162 (JP); The School Corporation Kansai University, Suita-shi, Osaka 564-8680 (JP)
(72) Inventor: AOYAGI, Seiji, Suita-shi, Osaka 564-8680 (JP); MATSUMOTO, Hajime, Inazawa-shi, Aichi 492-8162 (JP)
(74) Representative: Grünecker Patent- und Rechtsanwälte PartG mbB
(86) International application number: PCT/JP2018/020094
(87) International publication number: WO 2019/224991

(56) References cited:
- EP-A2- 1 764 047
- WO-A1-02/100273
- WO-A2-2004/091693
- US-A- 5 951 493
- US-A1- 2005 010 134
- US-B2- 8 057 404

## Description

### Technical Field

The present invention relates to a device according to the preamble of claim 1. Such a device is known from EP 1 764 047 A2. The disclosure herein relates generally to a device for blood collection and in particular to a device for collecting a tiny amount of blood from a blood capillary.

### Background Art

Some patients with blood-related disorders, such as diabetics, require routinely sampling and testing their own blood so as to know and appropriately manage their current blood conditions by using proper medicines such as insulin. As such tests nowadays require only tiny amounts of blood, blood is collected from neither veins nor arteries but blood capillaries in finger tips. A patient can, by himself or herself, take a lancet to lance the skin of the patient's finger into the capillary bed and extract a small drop of blood onto a test strip or into a tube. Then the collected blood is used in blood tests. This procedure is somewhat troublesome.

Some devices have been proposed to facilitate or partly automate this procedure. The patent literatures as indicated below disclose related arts. A further relevant prior art is described in EP 1 764 047 A2.

### Citation List

### Patent Literature

PTL 1: United States Patent Serial 8,012,104 B2
PTL 2: United States Patent Serial 8,333,715 B1
PTL 3: United States Patent Serial 9,782,114 B2

### Summary of Invention

A patient uses any of such devices to make on his/her finger a nick or cut large enough to cause spontaneous bleeding. After the blood collection, the patient has to take care of the finger that may keep bleeding gradually. Thus, even with these devices, the blood collection procedure is still troublesome and often causes the patient some pain.

Use of a very thin (around 200 microns in diameter for example) medical needle seems to reduce pain but is found not to be as effective as expected. The present inventor had studied microscopic phenomena in skin tissues around stuck needles and discovered that punctured with even a very thin needle considerably deforms a skin and hypodermic tissues and this deformation dominantly causes pain and as well bars blood flow because blood capillaries are squeezed. The present inventor had further discovered that use of a cannula with some particular motion such as reciprocating or intermittent spin can solve these problems. The device disclosed herein has been devised in light of these discoveries.

The present invention provides a device having the features defined in claim 1, further preferred embodiments are defined in the dependent claims.

### Brief Description of Drawings

[fig.1] FIG. 1 is an elevational view of a blood collection device fixed to a support according to an embodiment.
[fig.2] FIG. 2 is a sectional view of the blood collection device.
[fig.3] FIG. 3 is a sectional view of the blood collection device around a rotor and flow paths.
[fig.4A] FIG. 4A is a cross sectional view of the blood collection device taken from the line VIA-VIA of FIG. 3.
[fig.4B] FIG. 4B is a cross sectional view of a blood collection device according to a modified embodiment.
[fig.4C] FIG. 4C is a sectional view of a blood collection device according to another modified embodiment.
[fig.5] FIG. 5 is a sectional view of a blood collection device according to still another modified embodiment.
[fig.6] FIG. 6 is a sectional view of a sampling tube used in combination with the device.
[fig.7A] FIG. 7A is an enlarged plan view of the sampling tube, which mainly shows a cannula section at the tip end of the tube.
[fig.7B] FIG. 7B is a sectional view of the sampling tube, which is perpendicular to the plan shown in FIG. 7A.
[fig.8] FIG. 8 is an oblique plan view of a cannula section according to a modified example.
[fig.9] FIG. 9 is a sectional view of a sampling tube with a bulb section.

### Description of Embodiments

Exemplary embodiments will be described hereinafter with reference to the appended drawings.

Throughout the following description and appended claims, the term "axis" is defined and used as a rotational axis of a sampling tube unless otherwise stated. Accordingly, the terms "axial" and "axially" are defined and used in relation to the direction of the axis in this definition.

A device 1 according to any of the embodiments disclosed herein is generally usable for collecting a tiny amount of blood from a blood capillary but may be used for any other purposes, such as blood collection from a vein or artery, collection of any other body fluids or tissues, injection or infusion of a medicine, or placement of tiny devices on or in a body.

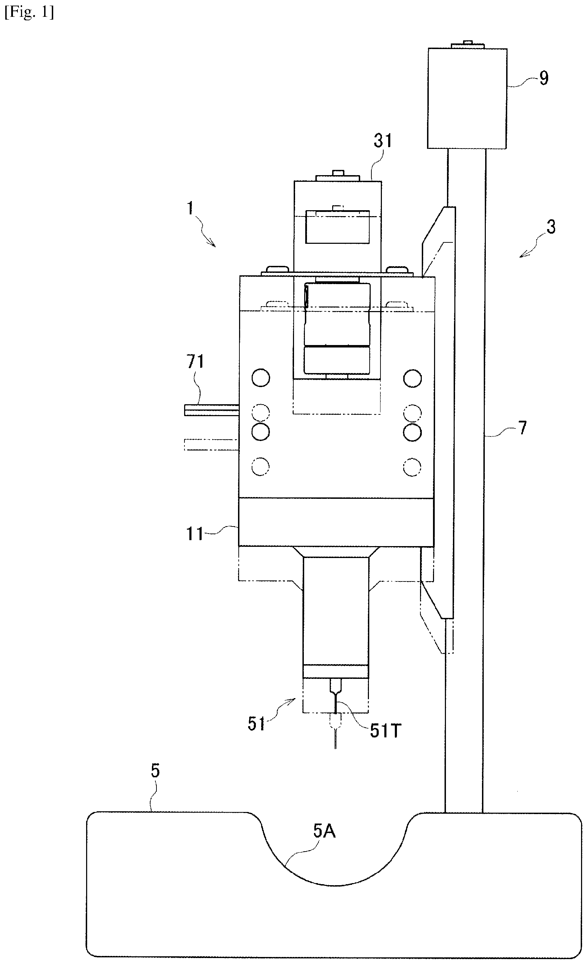

Although any hand-held operation is possible, the device 1 is preferably secured to a stable support 3 as shown in FIG. 1 and then used. The support 3 exemplarily shown in FIG. 1 is provided with a pedestal 5 and a post 7 vertically standing on and secured to the pedestal 5. The pedestal 5 is, when in use, placed on a desk or table or on any stable article. The pedestal 5 may be provided with a finger rest 5A in a shape of a depression on the pedestal for example.

The support 3 may be provided with an actuator 9 for automatically and controllably moving the device 1 closer to and away from the finger on the rest 5A. The actuator 9 may be constituted of an electric motor and a linear motion mechanism such as a ball screw or a rack-and-pinion. A linear motor system is alternatively applicable thereto. In any way, the motor may be electrically connected to a controller or a computer system via a wired or wireless network for the purpose of control or automation.

A sampling tube 51 having a pointed cannula section 51T for blood collection is housed in a body 11 of the device 1 with exposing the pointed cannula section 51T out of the head of the body 11. The sampling tube 51 is generally a capillary tube for sucking and holding a tiny amount of blood although details will be described later.

The device 1 is provided with a rotary device for setting the sampling tube 51 in reciprocating or intermittent spin motion about the spin axis and a fluid pressure source for applying a negative pressure to the hollow in the sampling tube. Either or both of them may be built in the device 1 but may be alternatively provided as exterior auxiliaries. A motor 31 is a built-in actuator for rotating the sampling tube 51. An adapter tube 71 exemplarily illustrated in FIG. 1 is adapted for coupling with an exterior pressure source but can be omitted if the pressure source is built in the device 1.

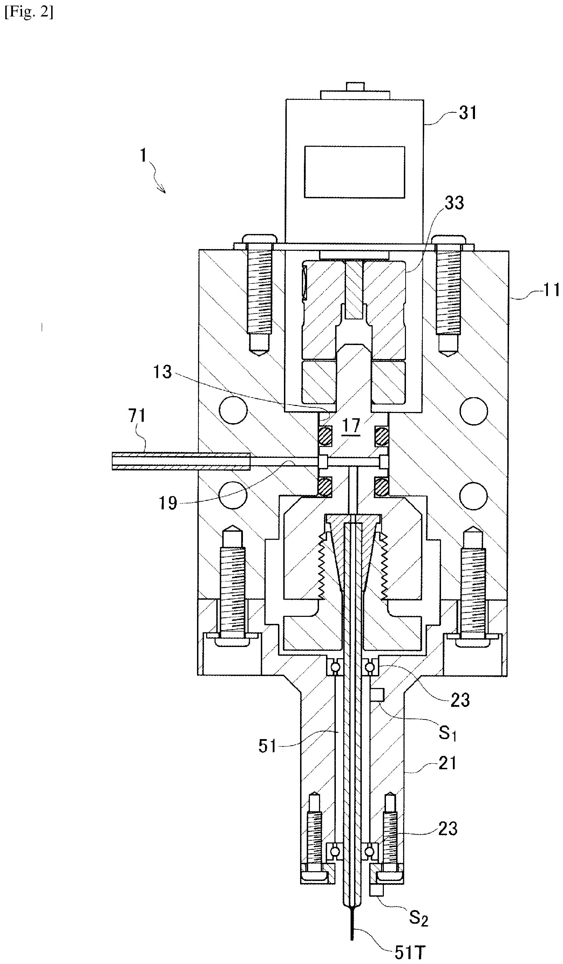

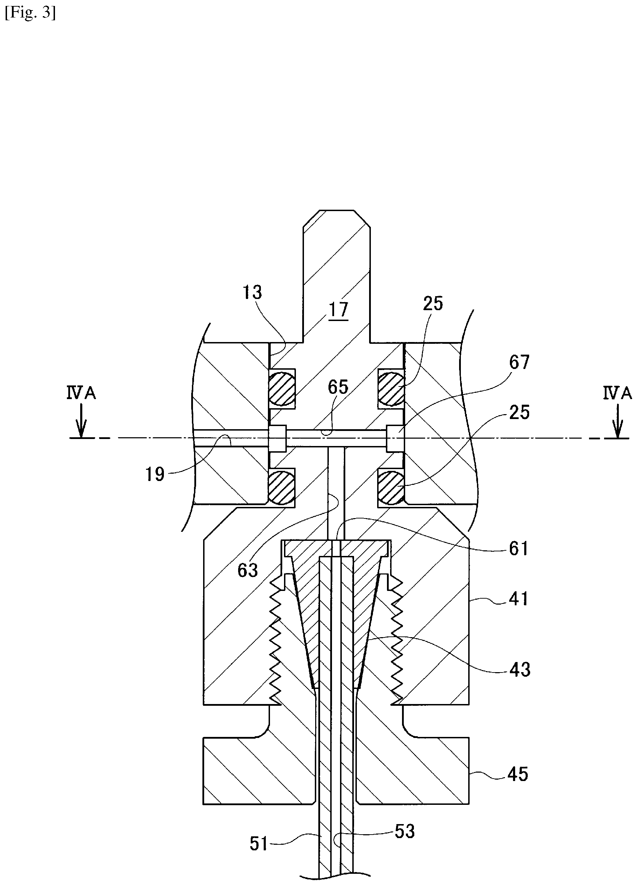

Referring to FIGs. 2 and 3 in combination with FIG. 1, the device 1 is generally comprised of the body 11 defining a bore 13, a rotor 17 fitting in the bore 13 and rotatable about the axis, and flow paths 61, 63, 65, 67 and 19 as intermediaries for fluid communication between the sampling tube 51 and the pressure source.

The bore 13 is generally a cylindrical opening extending along the axis. The rotor 17 is at least partly so structured as to fit in the bore 13 and is thus allowed to spin in both directions about the axis. To establish fluid-tightness, sealing members such as O-rings 25 may be interposed between the body 11 and the rotor 17.

The rotor 17 defines the flow path 63 extending axially toward the sampling tube 51. The rotor 17 further defines the flow path 65 in fluid communication with the flow path 63 and extending generally radially. The radial flow path 65 opens on the side wall of the rotor 17. The flow path 19 in the body 11 opens on substantially but not necessarily the same plane as the opening of the flow path 65.

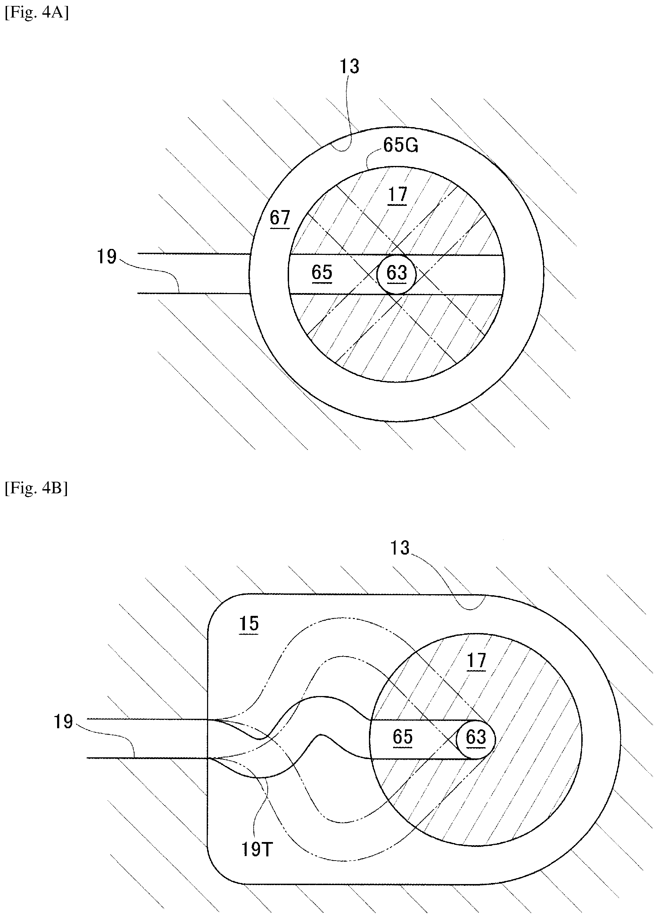

Referring to FIG. 4A in combination with FIGs. 1-3, on substantially but not necessarily the same plane as these opening formed is a peripheral groove 65G to form the flow path 67 between the body 11 and the rotor 17. This structure enables constant flow communication between the flow paths 19 and 65 even how the rotor 17 spins. While in the illustration the rotor 17 has the groove 65G, instead or additionally the body 11 may have a counterpart groove.

Any alternative structures may be instead used to establish constant flow communication. FIG. 4B exemplarily illustrates such a structure where a flexible tube 19T is connected to the flow paths 19 and 65 and a room 15 is held between the body 11 and the rotor 17 to allow the flexible tube 19T to meander therein.

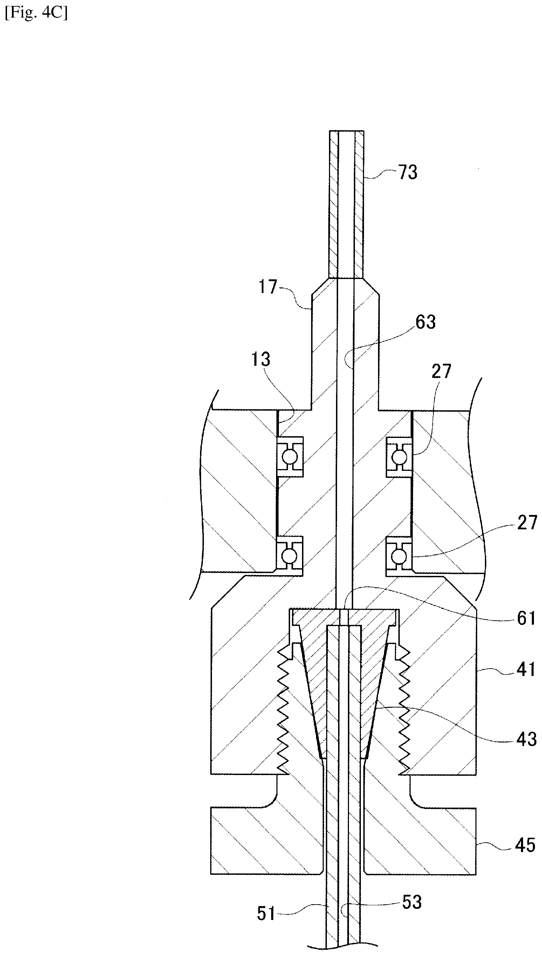

Still alternatively, the flow paths are not necessarily led out through the body 11 but may be directly led to the exterior. FIG. 4C exemplarily illustrates such a structure where the radial flow path 65 is omitted and the axial flow path 63 is further extended toward the top and connected to an upright adapter tube 73. Typically in this example, the O-rings 25 are unnecessary and instead ball bearings 27 may be used to facilitate smooth rotation. A hollow motor may be used in place of the aforementioned solid motor 31 and the adapter tube 73 may be led out through its hollow.

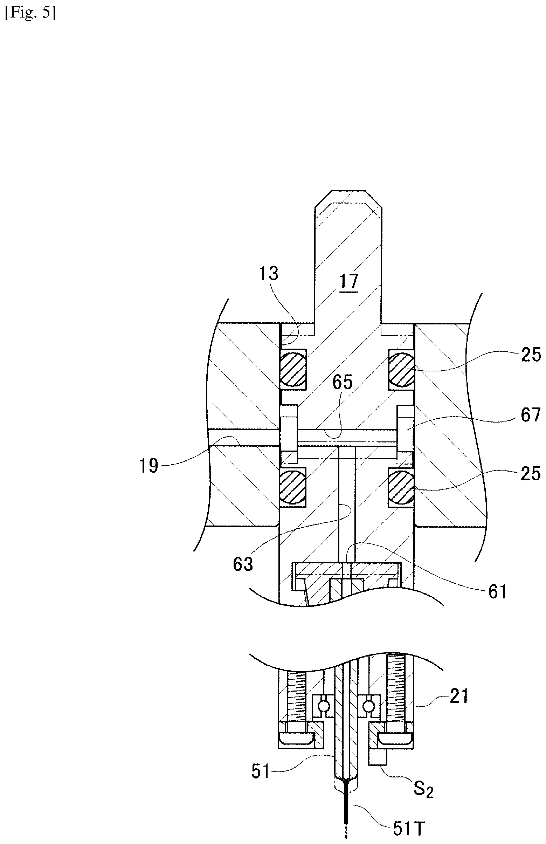

The rotor 17 is not required to be made immovable in the axial direction but may be so structured as to be movable in a relatively short distance in the axial direction as illustrated in FIG. 5. To establish constant fluid communication during the axial motion, the peripheral flow path 67 may be formed slightly wider in the axial direction. Further the device 1 may be provided with any actuator such as a piezo-electric actuator to create vibration or reciprocating axial movement in the rotor 17. Travel distance of this motion may be in the range of 0 to 0.1 mm and frequency may be in the range of 1 to 20 Hz but these examples are not limiting. This motion is conducted to the cannula section 51, thereby setting it in axially vibratory or reciprocating motion. Benefits by this motion will be described later.

Referring again to FIG. 2, the motor 31 is drivingly coupled with the rotor 17 via a coupling 33. Any DC motor, a servomotor or a stepping motor may be applied to the motor 31. The motor 31 or the coupling 33 may be provided with the piezo-electric actuator as described above.

When alternate current is applied to the motor 31, the shaft thereof is set into reciprocating spinning motion. When intermittent current is applied thereto, the shaft is set into intermittent spinning motion. Alternatively, any mechanism such as a link, a crank, a ratchet or a cam may be combined with the motor 31 in order to create intermittent or reciprocating spinning motion. In any way, the motion is transmitted via the rotor 17 to the sampling tube 51 and the cannula section S1T.

Referring mainly to FIG. 3, 4C or 5 in combination with FIG. 2, the device 1 is further provided with a fastening such as a nut 45 and the rotor 17 may be correspondingly provided with a skirt 41 so structured as to receive the nut 45. The fastening binds the sampling tube 51 to the rotor 17.

A sealing member such as a tapered sleeve 43 having a closed bottom with an opening 61 at its center may be held between the rotor 17 and the fastening in a state where the bottom faces the rotor 17 and the opening 61 is aligned with the flow path 63. When the fastening is tightened, the tapered sleeve 43 gets in close face-contact with the rotor 17, the sampling tube 51 and the nut 45, thereby establishing fluid tightness relative to the exterior but establishing fluid communication between a hollow 53 in the sampling tube 51 and the flow paths 61, 63, 65 and 19.

The sealing member such as the tapered sleeve 43 further provides benefit in facilitating incorporation or replacement of the sampling tube 51. Because, once the sampling tube 51 is inserted into the sealing member, the user can handle this combined body unitarily and the alignment and the fluid-tightness of the flow paths are automatically established when the fastening is tightened.

Referring mainly to FIG. 2, the body 11 is provided with a boss section 21 as a unitary body therewith or a detachable separate body. The boss section 21 extends along the axis so as to surround the sampling tube 51 but expose the cannula section 51T out of the boss section 21. This structure is adapted to support the sampling tube 51 substantially over the full length and therefore successfully supports the sampling tube 51 coaxially with the axis with preventing eccentric rotation. To realize smooth rotation, the boss section 21 may have two or more ball bearings 23, or any equivalent friction-reducing elements, and the bearings 23 are preferably apart in the axial direction from each other so as to prevent eccentric rotation.

The boss section 21 is preferably formed of a transparent or translucent material, such as glass, polystyrene, polyethylene and polypropylene, to allow visually checking collected blood, body fluid or tissue in the sampling tube 51.

Alternatively or additionally, the device 1 may be provided with a sensor S1 for detecting such a substance in the sampling tube 51. Applicable example is an optical sensor, a photoelectric sensor, a photocoupler or an inductive sensor but this list is not exhaustive.

It may be preferable to dispose the sensor S1 at a particular location where the sensor S1 can determine whether the amount of blood collected in the sampling tube 51 reaches a desired level. In this view, the sensor S1 may be disposed at a proper location relatively close to the tail end of the sampling tube 51 within the boss section 21. Further the device 1 may be provided with another sensor S2 around the tip end of the sampling tube 51 or the cannula section S1T to detect start of blood collection. Of course, the device 1 may be provided with still another sensor. In any way, the sensors may be connected to a controller or a computer system via a wired or wireless network and signals therefrom may be used as triggers for start and stop of blood collection.

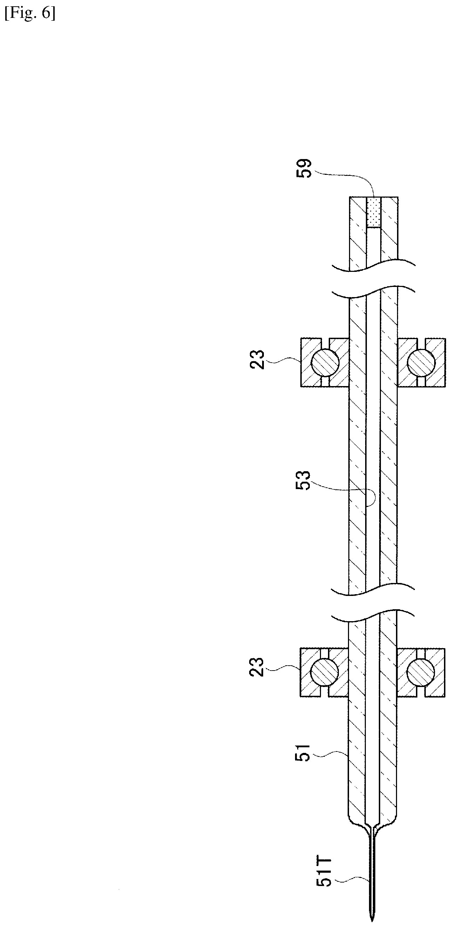

Referring to FIG. 6 in combination with FIG. 2, the sampling tube 51 is generally constituted of an elongated cylindrical tube and the cannula section S1T formed as a unitary body with or detachably attached to the tip end of the tube. The sampling tube 51 may be at least partly formed of a transparent or translucent material, such as glass, polystyrene, polyethylene and polypropylene, to allow visual check or optical sensing. The cannula section S1T may be nevertheless formed of any stiff or hard material such as glass or steel. Or, any biodegradable material such as polylactic acid or polyglycolide is applicable in light of environment-friendliness.

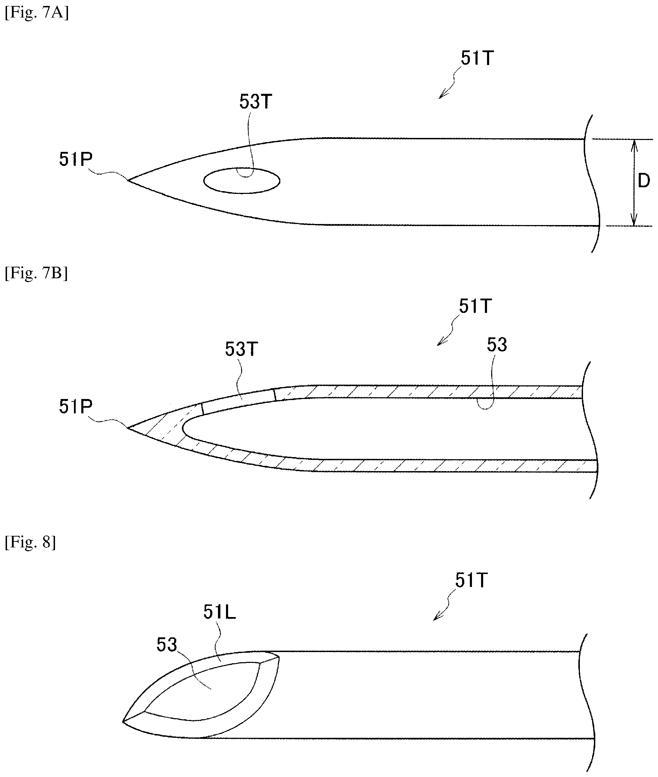

Referring to FIG. 7A in combination with FIG. 6, the cannula section S1T may be far thinner in diameter than the tube section and has a point 51P at its tip end for puncture. In general, the smaller the diameter D of the cannula section 51T is, the less pain a patient feels. However, an excessively thin cannula section may be likely to have buckling or damage. The diameter D may be less than 180 micrometers or preferably in the range of several tens to 120 micrometers.

Referring to FIGs. 7A and 7B, the hollow 53 in the sampling tube 51 extends to and reaches the point S1P and has an opening 53T around the point 51P. The point S1P may be hollow per se, or alternatively the opening 53T may be on the side wall close to the point S1P and in this case the point S1P may be solid. The cannula section S1T may be alternatively in any other form such as that of a medical needle as shown in FIG. 8 and may have bevels 51L sharply cut sideways to form a lancet shape.

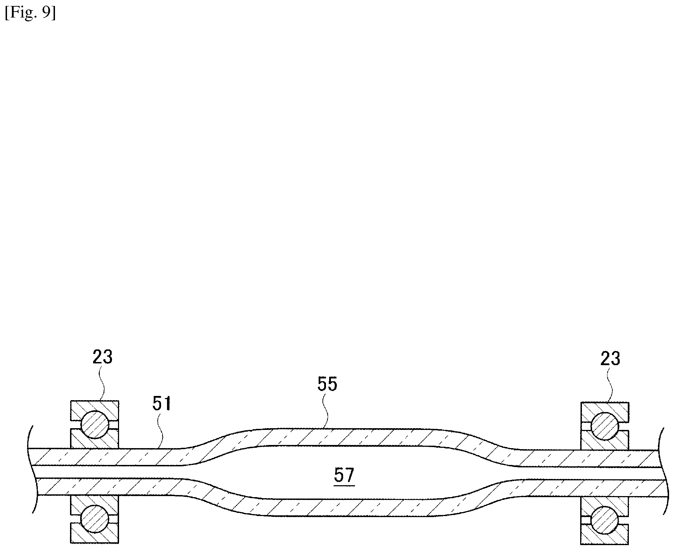

The sampling tube 51 aside from the cannula section S1T may be made uniform in diameter. This is beneficial in easy installation into the ball bearings 23. However, as shown in FIG. 9, the tube section may be partly made stout to have a bulb 55 that defines a greater cavity 57. This is beneficial in increasing the capacity.

The sampling tube 51 has the hollow 53 extending throughout the tube 51 and the cannula section S1T. The hollow 53 functions as both a medium for the negative pressure by the pressure source and a container for collected blood, body fluid or tissue. The hollow 53 opens at both the tip end and the tail end of the sampling tube 51, while the tail end may be filled with a breathable filter 59, which is beneficial in leakage prevention although allows fluid communication with the flow paths.

The device 1 is used in combination with an exterior or built-in pressure source that uses fluid, normally air, to apply negative or positive pressure. Applicable examples are a flexible hollow bulb such as a rubber bulb, a bellows pump or such a manual pump, a pre-pressurized or pre-depressurized air container and an electric air pump, but this list is not exhaustive. If the pressure source is arranged outside the device 1, it should be connected to the adapter tube 71 or 73. When the pressure source applies negative pressure to the flow paths, blood, body fluid or tissue is sucked and collected through the cannula section S1T into the hollow 53. The device 1 is instead usable for injection or infusion of any medical fluid into the body when the positive pressure is applied.

The procedure of blood collection will be exemplarily described hereinafter. This procedure is manually executable but may be partly or totally executed under control by any controller or computer system with a proper algorithm in accordance with the disclosure below.

A user (patient) can in advance give negative pressure to the pressure source if it is a manual tool and close its valve. First the user fixes the device 1 to the support 3 placed on any stable article and sets the user's finger on the rest 5A. Or, the user directly pinches the device 1 between fingers of one hand and puts the pointed cannula section 51T close to a finger of another hand.

The user next starts the motor 31, and the piezo-electric actuator if provided, to actuate the sampling tube 51 and further gets the device 1 down toward the finger to make the cannula section S1T puncture the skin thereof.

When the point S1P successfully catches any of blood capillaries, blood starts flashing up in the cannula section S1T as capillary force acts on the blood. Then the user can detect it by visually or by the sensor S2 checking the flashing blood in the cannula section S1T.

The user next stops the motor 31 and sets the pressure source in operation to apply the negative pressure to the sampling tube 51 through the flow paths and thus takes up the blood into the sampling tube 51. As the amount of collected blood reaches a desired level, the user can soon detect it by visual check or by the signal from the sensor S1.

The user then stops sucking by closing the valve or shutting down the pressure source. The user next gets the device 1 away from the finger and detaches the sampling tube 51 therefrom. When a new tube is installed therein, the device 1 can be used again.

The reciprocating or intermittent spin of the cannula section 51T, and/or the axial vibratory or reciprocating motion if applied, promotes cannula intrusion into the skin and the hypodermic tissues and reduces deformation thereof. Such puncture does not bar blood flow in the blood capillary as the blood capillaries are not squeezed. The user is not required to overly cut the skin to cause bleeding for the purpose of blood collection. The negative pressure through the flow paths automatically takes up and collects the blood into the sampling tube. These factors are beneficial in reducing pain and facilitating blood collection.

### Industrial Applicability

A device facilitating blood collection and reducing pain is provided.

## Claims

1. A device (1) comprising:
a fluid pressure source;
a body (11) defining a bore (13) extending along an axis; and
a first flow path (63,65,67,19) in fluid communication with the fluid pressure source,
**characterized by** comprising:
a replaceable sampling tube (51) having a pointed cannula section (51T) and a hollow (53) for capillary blood collection;
a rotor (17) fitting in the bore (13) and being capable of spinning in both directions about the axis;
a boss section (21) extending along the axis and so dimensioned as to rotatably support the sampling tube (51) coaxially with the axis and expose the cannula section (51T) out of the boss section (21); and
a detachable fastening (45) binding the sampling tube (51) to the rotor (17) so as to set the sampling tube (51) into rotation concentric with the axis and establish fluid communication between the first flow path (63,65,67,19) and the hollow (53) of the sampling tube (51).

2. The device (1) of claim 1, further comprising:
two or more axially apart bearings (23) interposed between the boss section (21) and the sampling tube (51), whereby the sampling tube (51) is prevented from eccentric rotation.

3. The device of claim 1, further comprising:
a second flow path (61) within the rotor (17) extending at least partly along the axis and establishing fluid communication between the hollow (53) and the first flow path (63,65,67,19).

4. The device of claim 3, further comprising:
a seal (43) interposed between the fastening (45) and the rotor (17) for establishing fluid-tightness and fluid communication between the hollow (53) and the second flow path (61).

5. The device of claim 4, wherein the seal (43) comprises a tapered sleeve so dimensioned as to exert pressure on both the rotor (17) and the sampling tube (51).

## Patentansprüche

1. Vorrichtung (1), die umfasst:
eine Fluiddruck-Quelle;
einen Körper (11), der eine sich entlang einer Achse erstreckende Bohrung (13) aufweist; und
einen ersten Strömungsweg (63, 65, 67, 19) in Fluidverbindung mit der Fluiddruck-Quelle,
**dadurch gekennzeichnet, dass** sie umfasst:
eine austauschbare Probenentnahme-Röhre (51) mit einem spitzen Kanülenabschnitt (51T) und einem Hohlraum (53) für Kapillarblut-Entnahme;
einen Rotor (17), der in die Bohrung (13) passt und sich in beiden Richtungen um die Achse herum drehen kann;
einen Nabenabschnitt (21), der sich entlang der Achse erstreckt und so dimensioniert ist, dass er die Probenentnahme-Röhre (51) koaxial zu der Achse drehbar trägt und den Kanülenabschnitt (51T) an dem Nabenabschnitt (21) freilegt; sowie
eine abnehmbare Befestigung (45), die die Probenentnahme-Röhre (51) an den Rotor (17) bindet, um die Probenentnahme-Röhre (51) in zu der Achse konzentrische Drehung zu versetzen und Fluidverbindung zwischen dem ersten Strömungsweg (63, 65, 67, 19) und dem Hohlraum (53) der Probenentnahme-Röhre (51) herzustellen.

2. Vorrichtung (1) nach Anspruch 1, die des Weiteren umfasst:
zwei oder mehr axial beabstandete Lager (23), die zwischen dem Nabenabschnitt (21) und der Probenentnahme-Röhre (51) angeordnet sind, wodurch die Probenentnahme-Röhre (51) an exzentrischer Drehung gehindert wird.

3. Vorrichtung nach Anspruch 1, die des Weiteren umfasst:
einen zweiten Strömungsweg (61) innerhalb des Rotors (17), der sich wenigstens teilweise entlang der Achse erstreckt und Fluidverbindung zwischen dem Hohlraum (53) und dem ersten Strömungsweg (63, 65, 67, 19) herstellt.

4. Vorrichtung nach Anspruch 3, die des Weiteren umfasst:
eine Dichtung (43), die zwischen der Befestigung (45) und dem Rotor (17) angeordnet ist, um Fluiddichtigkeit und Fluidverbindung zwischen dem Hohlraum (53) und dem zweiten Strömungsweg (61) herzustellen.

5. Vorrichtung nach Anspruch 4, wobei die Dichtung (43) eine konische Hülse umfasst, die so dimensioniert ist, dass sie sowohl auf den Rotor (17) als auch auf die Probenentnahme-Röhre (51) Druck ausübt.

## Revendications

1. Dispositif (1) comprenant :
une source de pression de fluide ;
un corps (11) définissant un alésage (13) qui s'étend le long d'un axe ; et
un premier chemin d'écoulement (63, 65, 67, 19) en communication fluidique avec la source de pression de fluide,
**caractérisé en ce qu'**il comprend :
un tube d'échantillonnage remplaçable (51) comportant une section de canule pointue (51T) et une cavité (53) pour la collecte de sang capillaire ;
un rotor (17) s'insérant dans l'alésage (13) et capable de tourner dans les deux sens autour de l'axe ;
une section de bossage (21) qui s'étend le long de l'axe et dimensionnée de manière à supporter de manière rotative le tube d'échantillonnage (51) coaxialement à l'axe et à exposer la section de canule (51T) hors de la section de bossage (21) ; et
une fixation détachable (45) liant le tube d'échantillonnage (51) au rotor (17) de manière à mettre le tube d'échantillonnage (51) en rotation concentrique à l'axe et à établir une communication fluidique entre le premier chemin d'écoulement (63, 65, 67, 19) et la cavité (53) du tube d'échantillonnage (51).

2. Dispositif (1) selon la revendication 1, comprenant en outre :
au moins deux paliers éloignés axialement (23) interposés entre la section de bossage (21) et le tube d'échantillonnage (51), de manière à empêcher une rotation excentrique du tube d'échantillonnage (51).

3. Dispositif selon la revendication 1, comprenant en outre :
un deuxième chemin d'écoulement (61) à l'intérieur du rotor (17) qui s'étend au moins en partie le long de l'axe et établit une communication fluidique entre la cavité (53) et le premier chemin d'écoulement (63, 65, 67, 19).

4. Dispositif selon la revendication 3, comprenant en outre :
un joint (43) interposé entre la fixation (45) et le rotor (17) pour établir une étanchéité aux fluides et une communication fluidique entre la cavité (53) et le deuxième chemin d'écoulement (61).

5. Dispositif selon la revendication 4, dans lequel le joint (43) comprend un manchon conique dimensionné de manière à exercer une pression à la fois sur le rotor (17) et sur le tube d'échantillonnage (51).
